# EUROPÄISCHE PATENTSCHRIFT

(11) **EP 0 647 152 B1**
(45) Veröffentlichungstag und Bekanntmachung des Hinweises auf die Patenterteilung: **15.01.1997**
(21) Anmeldenummer: 93915744.2
(22) Anmeldetag: 29.06.1993
(51) Int. Cl.: A61N 5/06, A61B 17/36

(54) **SONDE ZUM ERWÄRMEN VON KÖRPERGEWEBE**
PROBE FOR HEATING BODY TISSUE
SONDE POUR CHAUFFER UN TISSU DU CORPS

(30) Priorität: 29.06.1992 DE 4221364
(43) Veröffentlichungstag der Anmeldung: 12.04.1995
(73) Patentinhaber: KAUFMANN, Raimund, D-40225 Düsseldorf (DE); SCHWARZMAIER, Hans-Joachim, D-40225 Düsseldorf (DE)
(72) Erfinder: KAUFMANN, Raimund, D-40225 Düsseldorf (DE); SCHWARZMAIER, Hans-Joachim, D-40225 Düsseldorf (DE)
(74) Vertreter: Schaumburg, Thoenes & Thurn
(86) Internationale Anmeldenummer: EP9301677
(87) Internationale Veröffentlichungsnummer: WO9400194

(56) Entgegenhaltungen:
- EP-A- 0 370 890
- EP-A- 0 439 629
- DE-A- 4 137 983
- US-A- 1 550 197
- US-A- 1 721 019
- US-A- 2 074 634
- US-A- 4 967 765

## Beschreibung

Die Erfindung betrifft eine Sonde zum Erwärmen von Körpergewebe gemäß dem Oberbegriff des Anspruchs 1.

Seit 1983 (Bown SG, World J. Surg, 1983, 7, 700-709) wird der Laser zur interstitiellen Tumortherapie benutzt. Das Ziel des Verfahrens ist eine kontrollierte und möglichst homogene Erwärmung ausreichend großer Gewebevolumina (Durchmesser > 2 cm) durch Einkopplung von Licht über Lichtleiter. Der Hauptvorteil des Verfahrens ist darin zu sehen, daß nur ein dünner Stichkanal (< 2,55 mm) als chirurgischer Zugang zum Tumor erforderlich ist. Mit diesem minimal invasiven Verfahren können insbesondere tiefliegende Tumoren behandelt werden, die sonst keiner Therapie zugänglich sind. Da Krebszellen als besonders wärmeempfindlich gelten (Lambert AR; JAMA; 1912, 59,2147-2148), sind zur Tumorzerstörung bereits relativ geringe Temperaturerhöhungen um etwa 7°C bis 9°C während weniger Minuten ausreichend. Voraussetzung einer erfolgreichen "interstitiellen Thermotherapie" ist daher, den Tumor bis in seine Randbezirke um mindestens diese Temperatur zu erwärmen.

Bei Verwendung konventioneller Lichtleiter zur Energieeinkopplung (Bare Fiber; ITT-Faser^{R} DE 38 13 227) entsteht jedoch ein unerwünschtes Temperaturprofil im Gewebe insofern, als die Temperatur mit zunehmender Gewebetiefe steil abfällt. Als Konsequenz treten therapeutisch relevante Temperaturerhöhungen nur in einem Umkreis von wenigen Millimetern Durchmesser um die Einkoppelstelle auf (Masters A, Bown SG, Las.Med. Sci; 1990, 5, 129-136; Svaasand LO, et al., Las.Med. Sci; 1990, 5, 121-128). Dies ist zur Behandlung der meisten klinisch relevanten Tumoren unzureichend. Einer Erhöhung der Strahlungsleistung sind enge Grenzen gesetzt, da dies nicht nur zu Gewebeverdampfung bzw. -verkohlung sondern in Folge auch zur Zerstörung der Faser selbst führt. Die damit auf wenige Watt begrenzte Leistungsabgabe aus einer Glasfaser (bare fiber) von typischerweise 600 µm Durchmesser schließt somit eine therapeutische Nutzung des Prinzips in der Praxis weitgehend aus.

Im Prinzip ließe sich dieses Problem in gewissen Grenzen durch Vergrößerung des Faserdurchmessers und der Auskoppelfläche abmildern. Dem steht jedoch der Wunsch nach einem möglichst geringen Außendurchmesser des Gesamtsystems entgegen.

Es hat auch nicht an Versuchen gefehlt, das Problem durch die Verwendung speziell gestalteter, sogenannter Diffusorelemente zu lösen. Diese sollen in der Regel einerseits die Leistungsdichte an der Auskoppelstelle reduzieren und andererseits eine gleichmäßige Raumwinkelverteilung der abgestrahlten Energie gewährleisten. Bekanntgeworden sind hohle kugel- oder zylinderförmige Diffusorelemente, deren Hohlräume zum Teil mit Glaspartikeln definierter Größe gefüllt sind (Heinze A, et al, SPIE; 1990, 1201, 304-312). Zur Transmission ausreichend hoher Strahlungsenergie muß die Auskoppelfläche jedoch so stark vergrößert werden, daß die Diffusorelemente in nicht annehmbarer Weise vergrößert werden. Erschwerend kommt hinzu, daß die üblicherweise verwendeten Streupartikel in Folge ihrer Eigenabsorption erheblich zur thermischen Belastung des Auskoppelstückes beitragen. Material zur isotropen Aufstreuung von Laserlicht (z.B. Star WM, et al, Photochem. Photobiol.; 1987, 46, 5, 619-624) sind für hohe Leistungsdichten an optischen Grenzflächen (> 7 kW/cm²) bisher nicht beschrieben worden. Darüber hinaus besteht bisher keine Möglichkeit, die Raumwinkelverteilung der Abstrahlung auf einfache Weise beliebig zu modifizieren. Ebenfalls ungelöst ist das Problem des steilen Temperaturabfalls im Gewebe selbst. Eine möglichst gleichmäßige Temperaturverteilung in einem Gewebevolumen ließe sich im Grundsatz dadurch erreichen, daß die Einkoppelflächen aktiv gekühlt werden. Dies bedeutet allerdings, daß ein erheblicher Teil der eingeleiteten Gesamtenergie über eine aktive Kühlung wieder abgeführt werden muß. Zur ausreichenden Erwärmung (Temperaturerhöhung um 7°C - 9°C) eines größeren Tumors (Durchmesser > 2 cm) muß infolgedessen die transmittierbare Laserleistung um mindestens eine Größenordnung erhöht werden. Damit stellt sich das Problem der Eigenabsorption des Diffusorelementes und die damit verbundene Zerstörschwelle in erhöhtem Maße. Bisher sind keine Diffusorelemente bekannt, welche die benötigte Gesamtleistung von in der Regel mehr als 30 W unter Berücksichtigung des hier erforderlichen, geringen Außendurchmessers übertragen können.

Aus der DE-A-41 37 983 ist eine Sonde der im Oberbegriff des Anspruchs 1 beschriebenen Art bekannt, die im wesentlichen die vorstehend genannten Nachteile hat.

Der Erfindung liegt die Aufgabe zugrunde, eine Sonde der eingangs genannten Art mit einem in seiner geometrischen Ausdehnung möglichst begrenzten Diffusor- und Auskoppelelement so zu gestalten, daß bei mittleren Laserleistungen von mindestens 30 W eine Zerstörung der Auskoppeleinheit sicher vermieden und eine möglichst homogene Temperaturerhöhung des zu erwärmenden Gewebes im Radius von mehr als 10 mm um die Sonde erreicht wird, wobei Temperaturen, die zur Verdampfung oder Verkohlung von Gewebe führen könnten, sicher vermieden werden.

Diese Aufgabe wird durch die im kennzeichnenden Teil des Anspruchs 1 angegebenen Merkmale gelöst.

Ein erstes wesentliches Merkmal der erfindungsgemäßen Lösung betrifft die Ausbildung des optischen Diffusorelementes. Dadurch, daß Streupartikel, die hoch reflektierend sind und somit eine extrem geringe Eigenabsorption haben, in einer optisch transparenten Matrix eingebettet werden, ist es nicht nur möglich, Leistungen von 30 - 100 W zu übertragen, sondern es kann auch eine beliebig wählbare Abstrahlcharakteristik erreicht werden. Trotz der hohen übertragbaren Leistung läßt sich der Außendurchmesser des Diffusorelementes dabei auf maximal 1,5 mm begrenzen. Als hoch reflektierende Materialteilchen werden vorzugsweise Partikel aus Bariumsulfat (Brechungsindex n = 1,6), Magnesiumoxid oder Aluminlumstaub verwendet. Diese Partikel haben eine vernachlässigbare Eigenabsorption. Ihre geringe Größe (< 1 µm) erlaubt eine hohe Partikelkonzentration in dem Basismaterial. Daher ist die notwendige Anzahl von Streuprozessen zur Aufstreuung des Laserlichtes in den gewünschten Raumwinkel (z.B. eine isotrope Streuung) trotz der geringen zur Verfügung stehenden geometrischen Abmessungen des Diffusorelementes sichergestellt.

Als hoch transparentes Basismaterial werden vorzugsweise Glas, glasähnliche Grundstoffe, transparente Klebstoffe oder Methylacrylate (Methylmetacrylat) mit einem Brechungsindex n = 1,4 verwendet. Das letztgenannte Material hat eine hohe Transmission im hier interessanten roten und nahen infraroten Spektralbereich. Die Abstrahlcharakteristik des Diffusorelementes kann, insbesondere bei der Verwendung von Kunststoffen als Basismaterial noch während des Produktionsvorganges durch Veränderung der Streupartikelkonzentration variiert werden. Darüber hinaus ist das Material vor dem Aushärten beliebig verformbar.

Das zweite wesentliche Merkmal der erfindungsgemäßen Lösung betrifft die Integration des Diffusorelementes in ein Kühlsystem, das aufgrund des gut wärmeleitenden Materials des Lichtaustrittsabschnittes des Hüllrohres auch das diesen umgebende Gewebe kühlt. Trotz des geringen zur Verfügung stehenden Gesamtdurchmessers der Sonde (< 2,5 mm) kann das Kühlsystem so leistungsfähig gestaltet werden, daß im fasernahen Umfeld eine Temperatursenkung erfolgt, die ausreicht, ein möglichst homogenes Temperaturprofil zu erzeugen. Hierzu müssen 80 % - 90 % der eingekoppelten Laserenergie über die Kühlung wieder abgeführt werden. Hierzu ist es zweckmäßig, Kühlmittel zu verwenden, die unter 0°C gekühlt werden können, bei niedriger Temperatur eine ausreichende Viskosität besitzen und eine hohe Wärmekapazität haben. Ein Kühlmittel dieser Art kann beispielsweise ein Gemisch aus einer 0,9 % Natriumchloridlösung und Alkohol bzw. Methanol sein.

Obwohl es zunächst widersinnig erscheint, 80 -90 % der eingekoppelten Laserenergie wieder durch Kühlung abzuführen, wird gerade durch diese Maßnahme erreicht, daß auch in noch einer relativ großen Entfernung von der Sonde Gewebe erwärmt werden kann, ohne daß Gewebe in unmittelbarer Nachbarschaft der Sonde verdampft oder verkohlt und ohne daß die Sonde dabei zerstört wird. Dadurch, daß ein Großteil der durch Wärmeleitung übertragbaren Wärmemengen durch das Kühlsystem wieder abgeführt wird, erfolgt die Erwärmung des die Sonde umgebenden Gewebes in erster Linie durch Strahlungsabsorption. Die effektive Temperaturverteilung in dem die Sonde umgebenden Gewebevolumen läßt sich als das Ergebnis zweier überlagerter Prozesse betrachten, wie dies später noch genauer erläutert wird. Erfindungsgemäß wird die Tatsache ausgenutzt, daß entsprechend der relativ hohen Eindringtiefe der Laserstrahlung bei Wahl geeigneter Wellenlängen die Raumzeitintegrale für die durch Strahlung zugeführte und die durch Wärmeleitung abgeführte Wärmemenge nicht spiegelsymmetrisch verlaufen. Zeichnet man diese Prozesse über der radialen Entfernung von der Sonde im Gewebe auf, so ergibt sich bei geeigneter Dimensionierung der Energiezu- bzw. Abfuhr ein Temperaturraumprofil, daß in erster Näherung vergleichsweise flach über relativ große Gewebevolumina verläuft. Durch optimale Gestaltung der Abstrahlcharakteristik in Verbindung mit laserlichtgeeigneten Wellenlängen und Verwendung eines leistungsfähigen Kühlsystems läßt sich somit das Temperaturraumprofil erfindungsgemäß so günstig gestalten, daß das effektiv durch Temperaturerhöhung zerstörte Tumorgewebevolumen um mindestens einen Faktor 3 - 4 erhöht wird, wobei gleichzeitig die unerwünschte Überhitzung der Sondenspitze vermieden wird.

Gemäß einer besonders bevorzugten Ausführungsform ist der Lichtleiter hohl ausgebildet und von einer Außenhülle mit Abstand umgeben, die mindestens im Bereich des Diffusorelementes transparent und gut wärmeleitend ist, wobei daß Diffusorelement porös ausgeführt ist, so daß der Hohlraum des Lichtleiters und der Zwischenraum zwischen diesem und der Außenhülle einen Durchflußkanal für das Kühlmittel bilden. Der besondere Vorteil dieser Lösung besteht darin, daß das Kühlmittel gleichzeitig als Lichtleiter dient. Dies erlaubt ein besonders platzsparenden Aufbau der Sonde.

Eine andere Lösung, die ebenfalls dem Bau einer durchmesserkleinen Sonde erlaubt, besteht darin, daß der Lichtleiter von einer Außenhülle mit Abstand umgeben ist, die mindestens im Bereich des Diffusorelementes transparent und gut wärmeleitend ist und die durch eine achsparallel gerichtete Membran in mindestens zwei Kanäle unterteilt ist, die im Bereich des freien Sondenendes miteinander in Verbindung stehen.

Gemäß einer dritten Ausführungsform ist vorgesehen, daß der Lichtleiter von zwei koaxialen Außenhüllen unter Bildung eines ersten Kanals zwischen dem Lichtleiter und der radial inneren Hülle sowie eines zweiten Kanales zwischen den beiden Hüllen umgeben ist, wobei die beiden Hüllen mindestens im Bereich des Diffusorelementes transparent und gut wärmeleitend sind und wobei die beiden Kanäle im Bereich des freien Sondenendes miteinander in Verbindung stehen. Diese Lösung erlaubt einen großen Kühlmitteldurchsatz. Bei allen Ausführungsformen können die den Lichtleiter umgeben Hüllen starr oder flexibel sein, wobei das Material der Außenhülle aus einem zug- und druckfesten, in weiten Bereichen temperaturbeständigen und mit Ausnahme des transparenten Bereiches schlecht wärmeleitenden Kunststoff bestehen kann.

Ist das Diffusorelement an der Spitze der Sonde angeordnet, so ist diese zweckmäßiger Weise von einer transparenten Kappe gebildet, welche das Diffusorelement, umspült von den Kühlmittel, aufnimmt. Im Hinblick auf einen optimalen Wärmetransport wird die Kappe selbst idealerweise aus einem Diamant gefertigt. Eine ebenfalls gut wärmeleitende Saphirkappe ist hinsichtlich der Eigenabsorption bei Wellenlängen mit hoher Eindringtiefe in biologisches Gewebe (roter und naher infraroter Spektralbereich) ungeeignet. Aus Kostengründen wird man in der Regel eine Kappe aus Quarzglas oder ähnlichen Gläsern fertigen.

Bei der hierdurch festgelegten Wärmeleitung ist die maximal ableitbare Wärmemenge nur noch durch die Temperaturdifferenz zwischen Gewebe und Kühlflüssigkeit (ΔT) bestimmt. Legt man beispielsweise eine Oberfläche der Kappe von 30 mm² und einer Glaswanddicke von 0,3 mm zugrunde, so ergibt sich für die Ableitung von z.B. 27 W eine minimal notwendige Temperaturdifferenz ΔT von etwa 20°C.

Eine Temperaturdifferenz zwischen Gewebe und Kühlmittel von ca. 20 bis 30° C reicht jedoch zur Zerstörung großer Gewebevolumina noch nicht aus. Die Temperatur an der Faser muß vielmehr so stark abgesenkt werden, daß das durch Wärmeableitung erzeugte Temperaturprofil einen möglichst hohen Energleeintrag durch Strahlung erlaubt. Daher sollte das fasernahe Gewebe in der Regel auf Werte unter 0° C gekühlt werden.

Das Kühlmittel kann eine Flüssigkeit oder ein Gas sein, wobei für das die Sonde durchfließende Kühlmittel ein geschlossener Kühlkreislauf vorgesehen sein kann, der über einen Wärmertauscher mit einem sekundären Kühlkreislauf in Verbindung steht. Zur Kühlung kann auch die Entspannung eines Gases im Bereich des Diffusorelementes genutzt werden.

Weitere Merkmale der Erfindung ergeben sich aus den weiteren Unteransprüchen und der folgenden Beschreibung, welche in Verbindung mit den beigefügten Zeichnungen anhand von Ausführungsbeispielen erläutert. Es zeigen:
- Figur 1: eine schematische Darstellung der Sonde zusammen mit der Laserlichtquelle und dem Kühlsystem
- Figur 2: einen schematischen, die Sondenachse enthaltenen Schnitt durch den spitzennahen Bereich der Sonde gemäß einer ersten Ausführungsform der Erfindung
- Figur 2a: einen schematischen Querschnitt entlang Linie a - a in Figur 2,
- Figur 2b: einen schematischen Querschnitt entlang Linie b - b in Figur 2
- Figuren 3, 3a,3b: den Figuren 2, 2a, 2b entsprechende Darstellungen einer zweiten Ausführungsform der Erfindung,
- Figuren 4, 4a, 4b: den Figuren 2, 2a, 2b entsprechende Darstellungen einer dritten Ausführungsform der Erfindung
- Figuren 5a, 5b, 5c: jeweils einen die Sondenachse enthaltenden Schnitt durch drei Ausführungsformen eines Diffusorelementes in schematischer Darstellung und
- Figur 6: eine graphische Darstellung von Temperaturraumprofilen im Gewebe.

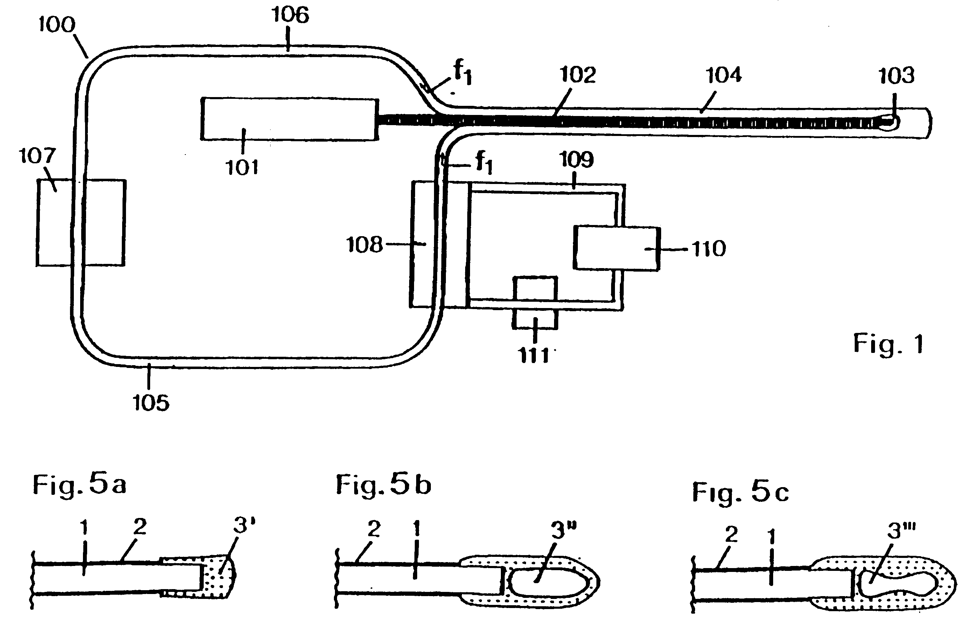

Figur 1 zeigt ein Lasersystem 100 zur Erwärmung großer Gewebevolumina und insbesondere zum Zerstören von Tumorgewebe durch Erwärmen. Das Lasersystem umfaßt eine Laserlichtquelle 101 mit einem angekoppelten Lichtleiter 102. Dieser endet in einem Diffusorelement 103, das von einem Kühlmittelkreislauf 104 mit einem Zulauf 105 und einem Ablauf 106 umschlossen ist. Eine Pumpe 107 bewirkt die Kühlmittelströmung in diesem geschlossenen Kühlmittelkreislauf in Richtung der Pfeile f1. Das Kühlmittel wird mittels eines Wärmetauschers 108 auf einer konstanten, gewünschten Temperatur gehalten. Der Wärmetauscher 108 liegt ferner in einem zweiten Kühlkreislauf 109 mit einer Pumpe 110, welche das Kühlmittel in dem Sekundärkreislauf 109 einem Kompressor 111 zuführt.

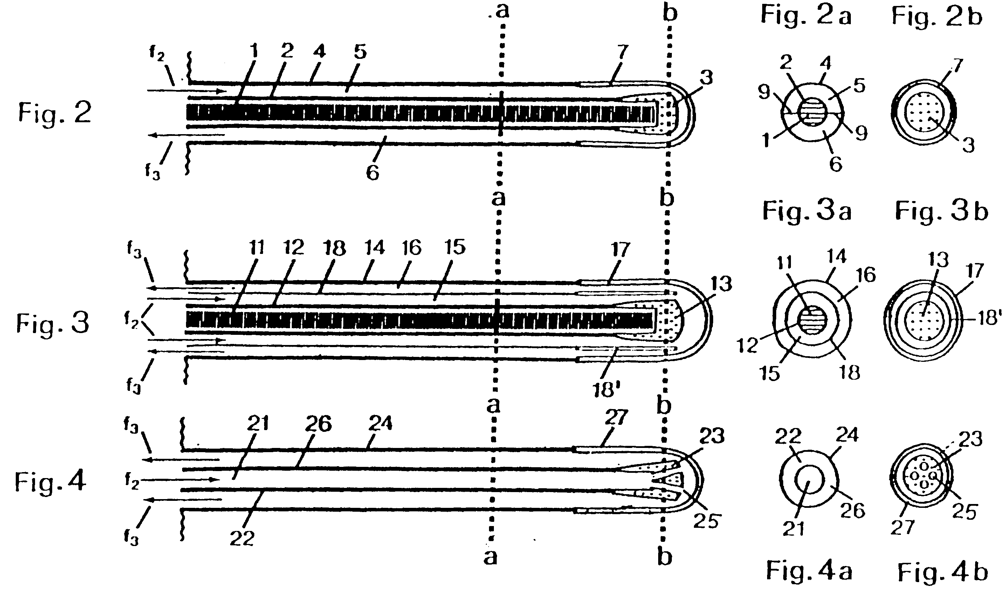

Eine erste Ausführungsform einer erfindungsgemäßen Lasersonde oder eines Laserkatheters ist im Längs- und Querschnitt in den Figuren 2, 2a und 2b gezeigt.

Der Lichtleiter 1 mit seinem Mantel 2 endet in einem Diffusorelement 3. Der Kühlmitteikreislauf wird mit Hilfe einer Außenhülle 4 realisiert. Der zwischen dem Fasermantel 2 und der Außenhülle 4 entstehende Hohlraum wird durch zwei achsparallel verlaufende Membranen 9 in einen Zulauf 5 und einen Ablauf 6 getrennt. Im Bereich des Diffusorelementes 3 endet die Außenhülle 4 in einer transparenten Kappe 7. Die Membranen 9 enden in diesem Bereich, so daß das Diffusorelement vom Kühlmittel umspült werden kann. Die Pfeile f2 und f3 zeigen die Strömungsrichtung des Kühlmittels im Zulauf bzw. Ablauf des Katheters.

Bei dem in den Figuren 3, 3a und 3b dargestellten Ausführungsbeispiel endet der Lichtleiter 11 mit seinem Mantel 12 ebenfalls in einem Diffusorelement 13. Der Kühlmittelkreislauf wird bei diesem Ausführungsbeispiel mittels zweier zusätzlicher Hüllen realisiert. Der Hohlraum 15 zwischen dem Fasermantel 12 und der radial inneren Hülle 18 dient hierbei als Zulauf. Der Hohlraum 16 zwischen der inneren Hülle 18 und der äußeren Hülle 14 bildet den Ablauf. Die Abschlußkappe 17 sowie der distale Teil 18' der inneren Hülle 18 sind aus transparentem Material gefertigt. Die Pfeile f2 und f3 zeigen wiederum die Strömungsrichtung des Kühlmittels im Zulauf bzw. Ablauf an.

Eine besonders bevorzugte Ausführungsform der Erfindung ist in den Figuren 4, 4a und 4b dargestellt. Der Lichtleiter 21 ist hier als Flüssigkeitslichtleiter ausgeführt und dient selbst als Kühlmittelzulauf. Das Kühlmittel bildet also gleichzeitig das Lichtleitermedium. Das Kühlmittel durchströmt dann das Diffusorelement 23 und tritt aus diesem durch Austrittsöffnungen 25 aus. Der Ablauf des Kühlmittels aus der transparenten Kappe 27 erfolgt durch einen Hohlraum 22, der von dem Fasermantel 26 und der äußeren Hülle 24 begrenzt wird. Auch hier zeichnen die Pfeile f2, f3 den Zulauf bzw. Ablauf des Kühlmittels an.

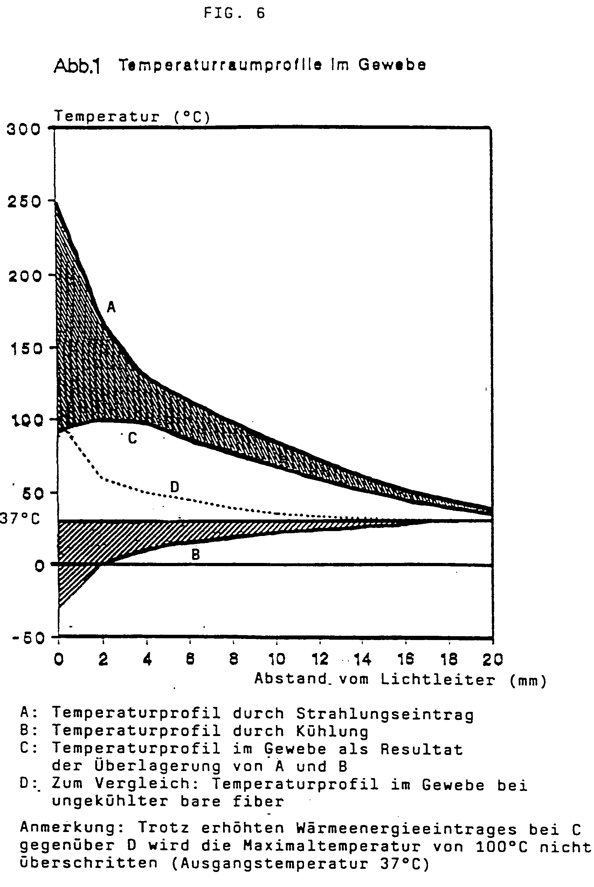

Die Figuren 5a, 5b und 5c zeigen jeweils das Ende eines Lichtleiters 1 mit seinem Mantel 2 und ein schematisch dargestelltes Diffusorelement 3', 3'', bzw. 3'''. Die Abbildungen zeigen, daß das Diffusorelement unterschiedlich geformt sein kann, um auf diese Weise unterschiedliche Abstrahlcharakteristiken hervorzurufen. Dabei kann auch die Verteilung der durch schwarze Punkte angedeuteten Streupartikel innerhalb des transparenten Basismaterials, in dem die Partikel eingelagert sind, inhomogen sein.

Figur 6 schließlich zeigt eine graphische Darstellung von Temperaturraumprofilen in einem Gewebe, in das die erfindungsgemäße Sonde eingeführt wurde, wobei die Temperatur (Ordinate) über dem radialen Abstand vom Lichtleiter (Abszisse) aufgetragen ist. Die Kurve A gibt das Temperaturprofil an, daß von dem Strahlungseintrag allein hervorgerufen wird. Die Kurve B zeigt das Temperaturprofil, das durch die Kühlung alleine hervorgerufen wird. Die Kurve C zeigt das Temperaturprofil, das sich als Resultat der Überlagerungen der Effekte gemäß A und B im Gewebe ergibt. Die gestrichelte Kurve D zeigt zum Vergleich das Temperaturprofil im Gewebe bei einer ungekühlten bare fiber.

Man erkennt, daß auch in unmittelbarer Nähe des Lichtleiters die Temperatur nicht über 100° C ansteigt. Dennoch ist auch in einem Abstand von 20mm von dem Lichtleiter noch eine merkliche Temperaturerhöhung vorhanden.

## Patentansprüche

1. Sonde zum Erwärmen von Körpergewebe durch Laserlicht hoher Intensität mit einem mit einer Laserlichtquelle (101) koppelbaren Lichtleiter (102; 1; 11; 21), an dessen in das Körpergewebe einführbarem Lichtaustrittsabschnitt ein Diffusorelement (103; 3; 13; 23) angeordnet ist, das aus einem für das Laserlicht hoch transparenten Basismaterial und in diesem eingelagerten hoch reflektierenden Materialteilchen besteht, und mit einer den Lichtleiter (102; 1; 11; 21) mit Abstand umgebenden Außenhülle (4; 14, 18; 24), die an einen Kühlmittelkreislauf (104) anschließbar ist, dadurch **gekennzeichnet**, daß die Außenhülle (4; 14, 18; 24) das Diffusorelement (103; 3; 13; 23) mit Abstand umgibt und mindestens im Bereich des Diffusorelementes (103; 3; 13; 23) aus einem transparenten und gut wärmeleitenden Material besteht.

2. Sonde nach Anspruch 1, dadurch **gekennzeichnet**, daß das hoch reflektierende Material aus der Bariumsulfat, Magnesiumoxid und Aluminiumstaub umfassenden Gruppe ausgewählt ist.

3. Sonde nach Anspruch 1 oder 2, dadurch **gekennzeichnet,** daß das für das Laserlicht hoch transparente Material aus der Glas, glasähnliche Kunststoffe, transparente Klebstoffe und Methylacrylate umfassenden Gruppe ausgewählt ist.

4. Sonde nach einem der Ansprüche 1 bis 3, dadurch **gekennzeichnet,** daß das hoch reflektierende Material in dem transparenten Material homogen verteilt ist.

5. Sonde nach einem der Ansprüche 1 bis 4, dadurch **gekennzeichnet**, daß das hoch reflektierende Material in dem transparenten Material in Abhängigkeit einer gewünschten Abstrahlcharakteristik inhomogen verteilt ist.

6. Sonde nach einem der Ansprüche 1 bis 5, dadurch **gekennzeichnet,** daß das Diffusorelement so geformt ist, daß eine mindestens annähernd kugelförmige Abstrahlcharakteristik entsteht.

7. Sonde nach einem der Ansprüche 1 bis 5, dadurch **gekennzeichnet,** daß das Diffusorelement so geformt ist, daß eine mindestens annähernd zylinderförmige Abstrahlcharakteristik entsteht.

8. Sonde nach einem der Ansprüche 1 bis 7, dadurch **gekennzeichnet,** daß der Lichtleiter (21) hohl ausgebildet ist und daß das Diffusorelement (23) porös ausgeführt ist derart, daß der Hohlraum des Lichtleiters (21) und der Zwischenraum zwischen diesem und der Außenhülle (24) einen Durchflußkanal (22) für das Kühlmittel bilden.

9. Sonde nach einem der Ansprüche 1 bis 7, dadurch **gekennzeichnet,** daß die Außenhülle (4) durch eine achsparallel gerichtete Membran (9) in mindestens zwei Kanäle (5, 6) unterteilt ist, die im Bereich des freien Sondenendes miteinander in Verbindung stehen.

10. Sonde nach einem der Ansprüche 1 bis 7, dadurch **gekennzeichnet,** daß der Lichtleiter (11) von zwei koaxialen Außenhüllen (18, 14) unter Bildung eines ersten Kanals (15) zwischen dem Lichtleiter (11) und der radial inneren Hülle (18) sowie eines zweiten Kanals (16) zwischen den beiden Hüllen (18, 14) umgeben ist, wobei die beiden Hüllen (18, 14) mindestens im Bereich des Diffusorelementes (13) transparent und gut wärmeleitend sind und wobei die beiden Kanäle (15, 16) im Bereich des freien Sondenendes miteinander in Verbindung stehen.

11. Sonde nach einem der Ansprüche 8 bis 10, dadurch **gekennzeichnet**, daß die den Lichtleiter (102; 1; 11; 21) umgebende Außenhülle (4; 14, 18; 24) bzw. Außenhüllen starr sind.

12. Sonde nach einem der Ansprüche 8 bis 10, dadurch **gekennzeichnet**, daß die den Lichtleiter (102; 1; 11; 21) umgebende Außenhülle (4; 14, 18; 24) bzw. Außenhüllen flexibel sind.

13. Sonde nach einem der Ansprüche 1 bis 12, dadurch **gekennzeichnet**, daß das Kühlmittel auch bei Temperaturen unterhalb 0°C eine niedrige Viskosität besitzt.

14. Sonde nach Anspruch 13, dadurch **gekennzeichnet**, daß das Kühlmittel ein Gemisch aus einer 0,9 %-igen Natriumchloridlösung und Alkohol bzw. Methanol ist.

15. Sonde nach einem der Ansprüche 1 bis 14, dadurch **gekennzeichnet**, daß im Bereich des Diffusorelementes ein Temperatursensor angeordnet ist.

16. Sonde nach einem der Ansprüche 1 bis 15, dadurch **gekennzeichnet,** daß die die Sonde bildenden Materialien kernspinkompatibel sind.

17. Sonde nach einem der Ansprüche 1 bis 16, dadurch **gekennzeichnet,** daß sie in Form eines Katheters ausgeführt ist, dessen distales Ende mit einer zum Einstechen in solides Gewebe geeigneten Spitze versehen ist.

18. Sonde nach einem der Ansprüche 1 bis 17, dadurch **gekennzeichnet**, daß der Kühlmitteikreislauf (104) für das die Sonde durchströmende Kühlmittel geschlossen ist und über einen Wärmetauscher (108) an einen Sekundärkreislauf (109) angeschlossen ist.

19. Sonde nach einem der Ansprüche 1 bis 18, dadurch **gekennzeichnet**, daß das gut wärmeleitende Material der Außenhülle (4; 14, 18; 24) Diamant oder Quarzglas ist.

## Claims

1. A probe for heating body tissue by high-intensity laser light, said probe comprising a light guide (102; 1; 11; 21) which can be coupled to a laser light source (101), and a diffusor element (103; 3; 13; 23) arranged at the light exit portion of said light guide adapted to be introduced into the body tissue, wherein said diffusor element is made of a base material highly transparent to the laser light and highly reflective material particles embedded therein, said probe further comprising an outer sleeve (4; 14, 18; 24) which surrounds the light guide (102; 1; 11; 21) at a distance and which can be connected to a coolant circuit, **characterized** in that said outer sleeve (4; 14, 18; 24) surrounds said diffusor element (103; 3; 13; 23) at a distance and is made of a material which is transparent and has good heat conductivity at least in the area of said diffusor element (103; 3; 13; 23).

2. A probe according to claim 1, **characterized** in that said highly reflective material is selected from the group comprising barium sulphate, magnesium oxide and aluminum powder.

3. A probe according to claim 1 or 2, **characterized** in that the material highly transparent to the laser light is selected from the group comprising glass, glass-like plastics, transparent adhesives and methyl acrylates.

4. A probe according to one of claims 1 to 3, **characterized** in that said highly reflective material is homogeneously distributed in the transparent material.

5. A probe according to one of claims 1 to 4, **characterized** in that said highly reflective material is distributed non-homogeneously in said transparent material depending on a desired radiation characteristic.

6. A probe according to one of claims 1 to 5, **characterized** in that said diffusor element is shaped such that the radiation characteristic produced is at least approximately spherical.

7. A probe according to one of claims 1 to 5, **characterized** in that said diffusor element is shaped such that the radiation characteristic produced is at least approximately cylindrical.

8. A probe according to one of claims 1 to 7, **characterized** in that said light guide (21) is hollow, and that said diffusor element (23) is porous such that the cavity of the light guide (21) and the space between said light guide and said outer sleeve (24) form a passageway (22) for the coolant.

9. A probe according to one of claims 1 to 7, **characterized** in that said outer sleeve (4) is divided into at least two channels (5, 6) by a membrane (9) extending in parallel to the axis, wherein said at least two channels communicate in the area of the free probe end.

10. A probe according to one of claims 1 to 7, **characterized** in that said light guide (11) is surrounded by two coaxial outer sleeves (18, 14), forming a first channel (15) between said light guide (11) and said radially inner sleeve (18), and a second channel (16) between the two sleeves (18, 14), wherein the two sleeves (18, 14) are transparent and have good heat conductivity at least in the area of the diffusor element (13), and wherein said two channels (15, 16) communicate in the area of the free probe end.

11. A probe according to one of claims 8 to 10, **characterized** in that the outer sleeve(s) (4; 14, 18; 24) surrounding the light guide (102; 1; 11; 21) is (are) rigid.

12. A probe according to one of claims 8 to 10, **characterized** in that the outer sleeve(s) (4; 14, 18; 24) surrounding the light guide (102; 1; 11; 21) is (are) flexible.

13. A probe according to one of claims 1 to 12, **characterized** in that the coolant has a low viscosity even at temperatures below 0°C.

14. A probe according to claim 13, **characterized** in that said coolant is a mixture of a 0.9% sodium chloride solution and alcohol or methanol.

15. A probe according to one of claims 1 to 14, **characterized** in that a temperature sensor is arranged in the area of the diffusor element.

16. A probe according to one of claims 1 to 15, **characterized** in that the materials forming the probe are nuclear magnetic resonance-compatible.

17. A probe according to one of claims 1 to 16, **characterized** in that it is designed as a catheter which has a tip adapted for puncturing solid tissue at its distal end.

18. A probe according to one of claims 1 to 17, **characterized** in that the coolant circuit (104) for the coolant flowing through the probe is closed and is connected to a secondary circuit (109) via a heat exchanger (108).

19. A probe according to one of claims 1 to 18, **characterized** in that said material of the outer sleeve (4; 14, 18; 24) having good heat conductivity is diamond or quartz glass.

## Revendications

1. Sonde destinée à chauffer des tissus corporels par une lumière laser de haute intensité, comprenant un guide de lumière (102 ; 1 ; 11 ; 21) pouvant être accouplé à une source de lumière laser (101), dont un segment de sortie de lumière peut être introduit dans les tissus corporels, et comporte un élément diffuseur (103 ; 3 ; 13 ; 23) qui est composé d'une matière de base à haute transparence pour la lumière laser et de particules de matière haute réfléchissante noyées dans cette matière de base, et une enveloppe extérieure (4 ; 14 ; 18 ; 24) qui entoure à distance le guide de lumière (102 ; 1 ; 11 ; 21), enveloppe qui peut être raccordée à un circuit d'agent de refroidissement (104), caractérisée en ce que l'enveloppe extérieure (4 ; 14, 18 ; 24) entoure à distance l'élément diffuseur (103 ; 3 ; 13 ; 23) et est en un matériau transparent et bon conducteur de la chaleur au moins dans la région de l'élément diffuseur (103 ; 3 ; 13 ; 23).

2. Sonde selon la revendication 1, caractérisée en ce que la matière hautement réfléchissante est choisie dans le groupe qui comprend le sulfate de baryum, l'oxyde de magnésium et la poudre d'aluminium.

3. Sonde selon la revendication 1 ou 2, caractérisée en ce que la matière hautement transparente pour la lumière laser est choisie dans le groupe qui comprend le verre, les matières plastiques analogues au verre, les matières adhésives transparentes et les méthilacrylate.

4. Sonde selon une des revendications 1 à 3, caractérisée en ce que la matière hautement réfléchissante est répartie de façon homogène dans la matière transparente.

5. Sonde selon une des revendications 1 à 4, caractérisée en ce que la matière hautement réfléchissante contenue dans la matière transparente est répartie de façon inhomogène en fonction d'une caractéristique d'émission de rayonnement désirée.

6. Sonde selon une des revendications 1 à 5, caractérisée en ce que l'élément diffuseur est conformé de manière qu'il s'établisse une caractéristique de rayonnement au moins approximativement sphérique.

7. Sonde selon une des revendications 1 à 5, caractérisée en ce que l'élément diffuseur est conformé de manière à établir une caractéristique de rayonnement au moins approximativement cylindrique.

8. Sonde selon une des revendications 1 à 7, caractérisée en ce que le guide de lumière (21) est creux et en ce que l'élément diffuseur (23) est de configuration poreuse, de telle manière que la qualité du guide de lumière (21) et l'espace entre ce guide et l'enveloppe extérieure (24) forment un canal de passage (22) pour le milieu de refroidissement.

9. Sonde selon une des revendications 1 à 7, caractérisée en ce que l'enveloppe extérieure (4) est divisée par une membrane (9) orientée parallèlement à l'axe en au moins deux canaux (5, 6) qui sont en communication entre eux dans la région de l'extrémité libre de la sonde.

10. Sonde selon une des revendications 1 à 7, caractérisée en ce que le guide de lumière (11) est entouré de deux enveloppes extérieures coaxiales (18, 14) avec formation d'un premier canal (15) entre le guide de lumière (11) et l'enveloppe radialement intérieure (18), ainsi qu'un deuxième canal (16) compris entre les deux enveloppes (18, 14), les deux enveloppes (18, 14) étant transparentes et bonnes conductrices de la chaleur au moins dans la région de l'élément diffuseur (13) et les deux canaux (15, 16) étant en communication entre eux dans la région de l'extrémité libre de la sonde.

11. Sonde selon une des revendications 8 à 10, caractérisée en ce que l'enveloppe extérieure (4 ; 14, 18; 24) ou les enveloppes extérieures qui entoure(nt) le guide de lumière (102 ; 1 ; 21) sont rigides.

12. Sonde selon une des revendications 8 à 10, caractérisée en ce que l'enveloppe extérieur (4 ; 14, 18 ; 24) ou les enveloppes extérieures qui entoure(nt) le guide de lumière (102 ; 1 ; 11 ; 21) sont flexibles.

13. Sonde selon une des revendications 1 à 12, caractérisée en ce que l'agent de refroidissement possède une basse viscosité, même à des températures inférieures à 0°C.

14. Sonde selon la revendication 13, caractérisée en ce que l'agent de refroidissement est un mélange d'une solution de chlorure de sodium à 0,9 % et d'alcool ou de méthanol.

15. Sonde selon une des revendications 1 à 14, caractérisée en ce qu'un capteur de température est agencé dans la région de l'élément diffuseur.

16. Sonde selon une des revendications 1 à 15, caractérisée en ce que les matériaux qui forment la sonde sont compatibles en spin nucléaire.

17. Sonde selon une des revendications 1 à 16, caractérisée en ce qu'elle est réalisée sous la forme d'un cathéter dont l'extrémité distale est munie d'une pointe appropriée pour pouvoir être piquée dans des tissus massifs.

18. Sonde selon une des revendications 1 à 17, caractérisée en ce que le circuit de refroidissement (104) pour le milieu de refroidissement qui parcourt la sonde est fermé et est raccordé à un circuit secondaire (109) par l'intermédiaire d'un échangeur de chaleur (108).

19. Sonde selon une des revendications 1 à 18, caractérisée en ce que la matière bonne conductrice de la chaleur de l'enveloppe extérieure (4 ; 14, 18 ; 24) est un diamant ou un verre siliceux.
